# EUROPÄISCHE PATENTANMELDUNG

(11) **EP 3 354 294 A1**
(43) Veröffentlichungstag der Anmeldung: **01.08.2018**
(21) Anmeldenummer: 17153346.6
(22) Anmeldetag: 26.01.2017
(51) Int. Cl.: A61M 1/10

(54) **VORRICHTUNG ZUM ABSCHEIDEN VON KONDENSWASSER**

(71) Anmelder: Berlin Heart GmbH, 12247 Berlin (DE)
(72) Erfinder: Arslan, Nedim, 10717 Berlin (DE); Graichen, Kurt, 13189 Berlin (DE); Robbenmenke, Christian, 10965 Berlin (DE); Rodemerk, Aaron, 14482 Potsdam (DE)
(74) Vertreter: Pfenning, Meinig & Partner mbB

(57) **Zusammenfassung**

Die Erfindung betrifft eine Vorrichtung zum Abscheiden von Kondenswasser aus einem Betriebsgas eines pneumatischen, pulsatilen Systems, wobei die Vorrichtung einen Gasleitungsabschnitt (1) mit einem Gaseinlass (2) und einem ersten (3) und einem zweiten Gasauslass (4) aufweist. Die Vorrichtung zeichnet sich ferner dadurch aus, dass die Vorrichtung einen Kondensationsbehälter (5) zum Extrahieren des Kondenswassers aus dem Betriebsgas umfasst, der mit dem zweiten Gasauslass (4) verbunden ist, wobei ein Übergangsbereich vom zweiten Gasauslass (4) zum Kondensationsbehälter (5) eine Querschnittvergrößerung aufweist, und der Kondensationsbehälter (5) ein Mittel zum Abführen des extrahierten Kondenswassers aufweist. Die Erfindung betrifft weiterhin ein pneumatisches, pulsatiles System sowie ein Herzunterstützungssystem.

## Beschreibung

Die vorliegende Erfindung betrifft eine Vorrichtung zum Abscheiden von Kondenswasser aus einem Betriebsgas eines pneumatischen, pulsatilen Systems, insbesondere eines pneumatischen, pulsatilen Systems für eine Membranpumpe eines Herzunterstützungssystems.

Derartige Herzunterstützungssysteme sind zur links- bzw. rechtsseitigen oder beidseitigen Herzunterstützung vorgesehen, wenn das natürliche Herz auch unter medikamentöser Therapie oder durch invasive intensivmedizinische Maßnahmen nicht in der Lage ist, einen für den Patienten adäquaten Blutfluss und Blutdruck aufrecht zu erhalten. Diese Systeme bestehen jeweils aus einer oder zwei Membranpumpen (im Folgenden auch als Blutpumpen oder Herzpumpen bezeichnet) mit Antriebsschläuchen und Kanülen sowie einem Antriebssystem (pneumatisches, pulsatiles System) und sind für den Einsatz in klinischer und häuslicher Umgebung bestimmt. Ferner sind die Systeme für eine mittel- bis langfristige mechanische Unterstützung vorgesehen.

Die Blutpumpe besteht aus einer Blutkammer und einer Luftkammer, die durch eine mehrlagige flexible Membran aus Polyurethan voneinander getrennt sind. Ein Antriebsschlauch verbindet die Luftkammer der Blutpumpe mit dem Antrieb. Der vom pneumatischen Antrieb erzeugte Saug- bzw. Treibdruck bewegt die Membran, wodurch die Blutpumpe gefüllt und entleert wird. Das Blut fließt aus Atrium oder Ventrikel durch die Einlasskanüle in die Blutkammer der Blutpumpe und von dort durch die Gefäßkanüle in die Pulmonalarterie bzw. die Aorta. Klappen im Ein- bzw. Auslassbereich der Blutpumpe gewährleisten einen gerichteten Blutfluss. In dem pneumatischen Antrieb kommt es zum abwechselnden Aufbau von Unterdruck und Überdruck an der Membran der Blutpumpe, und damit zur Füllung und Entleerung der Blutpumpe. Über ein Ausgleichsventil wird die Luftmasse im Zylinder geregelt. Die Druckerzeugung kann auf verschiedene Weisen erfolgen. Beispielsweise kann ein Kolben über eine Spindel in einem Zylinder durch einen Motor hin und her bewegt werden. Durch diese Kolbenbewegung wird der abwechselnde Unter- und Überdruck erzeugt. Ein anderer Antriebsmechanismus ist so gestaltet, dass zwei Behälter durch entsprechende Kompressoren konstant mit Unter- bzw. Überdruck versorgt werden und ein spezielles Ventil eingesetzt wird, welches über einen Antriebsschlauch die Membranpumpe abwechselnd mit einem der beiden Behälter verbindet.

Bei der Kompression von Luft, beispielsweise in Kolbenverdichtern zur Drucklufterzeugung, kann Wasser in Form von Kondensat ausfallen. Ebenso kann Kondenswasser entstehen, wenn kalte Oberflächen mit warmer, feuchter Luft in Kontakt kommen. Beide Effekte treten auch in den oben beschriebenen pneumatischen, pulsatilen Systemen für Herzunterstützungssysteme auf. Die bisher bekannten pneumatischen Systeme verfügen über keinen zufriedenstellenden Mechanismus, um das entstehende Kondenswasser abzuführen.

In diesen pneumatischen, pulsatilen Systemen sammelt sich das Kondenswasser beispielsweise bevorzugt in den pneumatischen Schläuchen zwischen den Antriebskolben und der Membranpumpe . Kommt es zur Bildung von Kondenswasser, müssen die Schläuche dekonnektiert werden und das Wasser manuell abgelassen werden. Diese Umstände können den am Gerät befindlichen Patienten mitunter beunruhigen und belasten.

Bei manchen Systemen konnte in einigen Fällen Kondenswasser im Inneren des Antriebs beobachtet werden. In einer Zuleitung vom Hauptpneumatikstrang zu einem Drucksensor kann das Kondensat zu einer Funktionsbeeinträchtigung des Sensors führen.

Es sind konventionelle Kondenswasser-Abscheider erhältlich, welche vornehmlich in der Drucklufterzeugung eingesetzt werden. Diese Abscheider haben den Nachteil, dass ein großer Bauraum benötigt wird, dass einige Modelle zusätzliche Energie verbrauchen und dass eine potentielle Quelle für ungewollte Leckagen (bei Fehlfunktion) ins System integriert wird.

Ebenfalls bekannt sind passive Systeme, welche mit einem Schlauch arbeiten, der zwar luftdicht ist, Wasserdampf allerdings hindurch diffundieren lässt. Um eine Diffusion zu ermöglichen, sind jedoch hohe Differenzdrücke (ca. 4 - 5 bar) nötig.

Eine Aufgabe der vorliegenden Erfindung ist es daher, eine Vorrichtung zum Abscheiden von Kondenswasser bereitzustellen, welche die oben beschriebenen Probleme des Standes der Technik löst. Insbesondere ist es eine Aufgabe der vorliegenden Erfindung, eine Vorrichtung bereitzustellen, die eine effektive Kondenswasser-Abscheidung ermöglicht, keine zusätzliche Energie benötigt, sehr kompakt ist und ein sehr geringes Leckage- Risiko mit sich bringt. Eine weitere Aufgabe ist es, ein pneumatisches, pulsatiles System und ein Herzunterstützungssystem mit einer derartigen Vorrichtung bereitzustellen.

Die Aufgabe wird durch eine Vorrichtung gemäß Anspruch 1, durch ein pneumatisches, pulsatiles System gemäß Anspruch 14 und durch ein Herzunterstützungssystem gemäß Anspruch 15 gelöst. Vorteilhafte Weiterbildungen der erfindungsgemäßen Vorrichtung sind in den abhängigen Ansprüchen 2 bis 13 beschrieben.

Die erfindungsgemäße Vorrichtung zum Abscheiden von Kondenswasser aus einem Betriebsgas eines pneumatischen, pulsatilen Systems weist einen Gasleitungsabschnitt mit einem Gaseinlass und einem ersten sowie einem zweiten Gasauslass auf. Dieser Gasleitungsabschnitt ist an seinem Gaseinlass und seinem ersten Gasauslass mit einer Gasleitung zum Führen eines Betriebsgases, vorzugsweise von Luft, eines pneumatischen, pulsatilen Systems verbindbar, so dass das Betriebsgas über den Gaseinlass in den Gasleitungsabschnitt gelangen und über den ersten Gasauslass aus dem Gasleitungsabschnitt austreten kann.

Weiter umfasst die erfindungsgemäße Vorrichtung einen Kondensationsbehälter zum Extrahieren des Kondenswassers aus dem Betriebsgas, der mit dem zweiten Gasauslass des Gasleitungsabschnitts verbunden ist. Ein Übergangsbereich vom zweiten Gasauslass zum Kondensationsbehälter weist dabei eine Querschnittvergrößerung auf. Der Kondensationsbehälter weist ferner ein Mittel zum Abführen des extrahierten Kondenswassers auf.

Die Erfindung beruht auf zwei wesentlichen Prinzipien, welche in der oben beschriebenen Merkmalskombination die erwünschte Wirkung erzielen. Diese Prinzipien umfassen das Sammeln von Kondenswasser und das Abführen von Kondenswasser.

Um das Kondenswasser zu sammeln, wird ein thermodynamischer Effekt ausgenutzt, der nachfolgend beschrieben wird. Der pneumatische Antrieb erzeugt einen zyklischen Über- bzw. Unterdruck, welcher über die Gasleitung zu einer mit der Gasleitung verbundenen Membranpumpe geleitet wird. Beim Übergang von der Gasleitung zur Pumpe kommt es zu einer raschen Expansion des Betriebsgases, und es wird eine Abkühlung im Vergleich zur Umgebungstemperatur beobachtet. Dadurch kondensiert das in dem Betriebsgas gebundene Wasser bevorzugt an dieser Stelle. Der gleiche Effekt tritt beispielsweise auch auf, wenn Drucksensoren mit dem pneumatischen, pulsatilen System (auch als Pneumatik- oder Antriebsstrang bezeichnet) verbunden werden und dadurch Querschnittänderungen beim Übergang von der Gasleitung zum Drucksensor auftreten. Die Erfindung nutzt dieses Prinzip gezielt aus, indem der Übergangsbereich vom Gasauslass zum Kondensationsbehälter mit einer Querschnittvergrößerung ausgebildet wird. So wird das Kondenswasser im Kondensationsbehälter gesammelt und kann sich nicht an unerwünschten Stellen niederschlagen. In einem Experiment wurde bestätigt, dass das Prinzip funktionstüchtig ist und das Kondenswasser vornehmlich im dafür vorgesehenen Kondensationsbehälter entsteht.

Um das Kondenswasser abzuführen, weist der Kondensationsbehälter mindestens ein entsprechendes Mittel auf. In einer besonders vorteilhaften Ausgestaltung der Erfindung weist der Kondensationsbehälter hierzu ein wasserdurchlässiges Material, insbesondere einen semipermeablen Kunststoff, ein Fluorpolymer und/oder ein Sintermetall auf, durch welches das Wasser hindurch diffundieren kann und welches vorteilhafterweise gasundurchlässig ist. Als derartige halbdurchlässige Materialien sind Fluorpolymere in der technischen Anwendung bereits bekannt, erfordern zur Ableitung von Wasserdampf jedoch einen Luftdruck von ca. 4 -5 bar. Die Besonderheit der hier vorgestellten Ausgestaltung der Erfindung liegt darin, dass das Material permanent mit Wasser benetzt werden kann. In diesem Fall kann das Kondenswasser auch bei geringen Drücken abgeleitet werden. Die Wirksamkeit dieses Prinzips konnte experimentell ebenfalls bestätigt werden.

Die erfindungsgemäße Vorrichtung kann insbesondere in stationären und nicht-stationären pneumatischen Antrieben für Herzunterstützungssysteme eingesetzt werden, um eine Ansammlung von Kondenswasser zuverlässig zu verhindern. Die Vorrichtung arbeitet völlig selbstständig und weitgehend wartungsfrei, bietet somit also einen hohen Mehrwert für den Patienten. Es ist ferner denkbar, die Erfindung in weiteren Systemen einzusetzen, in denen pulsatile Druckverhältnisse herrschen und Kondenswasser abgeführt werden soll. Beispielsweise ist es denkbar, das System in Schläuchen für künstlich beatmete Patienten zu verwenden.

In einer vorteilhaften Ausgestaltung der Erfindung kann der Kondensationsbehälter eine Wandung aufweisen, die zumindest in einem Teilbereich des Kondensationsbehälters ein wasserdurchlässiges Material, insbesondere einen semipermeablen Kunststoff, ein Fluorpolymer und/oder ein Sintermetall enthält oder daraus besteht. Die Größe dieses Teilbereichs in Bezug auf die Größe der Oberfläche der Wandung ist dabei im Allgemeinen abhängig vom verwendeten Material und der abzuführenden Wassermenge. In einer bevorzugten Ausgestaltung der Erfindung beträgt die Größe des Teilbereichs mit dem wasserdurchlässigen Material etwa 5 cm².

In einer alternativen Ausgestaltung der Erfindung kann als Mittel zum Abführen des Kondenswassers ein Ventil oder eine Pumpe im Kondensationsbehälter angeordnet sein. Ferner wäre es denkbar, den Kondensationsbehälter über eine trennbare Kupplung mit dem zweiten Gasauslass zu verbinden.

Zur Verstärkung des Kondensationseffekts kann der Übergangsbereich vom zweiten Gasauslass zum Kondensationsbehälter vor der Querschnittvergrößerung eine Querschnittverengung aufweisen. Hierdurch wird das durch den Übergangsbereich strömende Betriebsgas vor der Expansion verdichtet. Die Verdichtung des Betriebsgases ermöglicht das Ausfallen zumindest eines Teils des im Betriebsgas gebundenen Wasserdampfes und führt somit zu einer Vergrößerung der aus dem Betriebsgas extrahierten Wassermenge. Dabei kann zwischen dem zweiten Gasauslass und dem Kondensationsbehälter insbesondere ein Leitungsverbindungsstück angeordnet sein, wobei ein minimaler Querschnitt des Leitungsverbindungsstücks kleiner ist als ein minimaler Querschnitt des zweiten Gasauslasses und/oder als ein maximaler Querschnitt des Kondensationsbehälters. Der Querschnitt des Leitungsverbindungsstücks zwischen dem zweiten Gasauslass und dem Kondensationsbehälter kann konstant sein. Besonders vorteilhaft ist es jedoch, wenn das Leitungsverbindungsstück eine Düse mit einem sich vom zweiten Gasauslass zum Kondensationsbehälter hin verengenden Querschnitt aufweist. Die Düse ermöglicht eine Verdichtung des Betriebsgases vor der Expansion im Kondensationsbehälter und somit eine Verbesserung der Abscheideleistung.

In einer weiteren vorteilhaften Ausgestaltung der Erfindung weist der Gasleitungsabschnitt einen ersten Gasleitungsabschnitt und einen zweiten Gasleitungsabschnitt auf, welche über in einer Linie verlaufende Verbindungsarme eines T-Stücks miteinander verbunden sind, und der zweite Gasauslass von dem Teil des T-Stücks gebildet wird, welcher weitgehend senkrecht zu den Verbindungsarmen abgezweigt ist. Die Verwendung eines T-Stücks vereinfacht die Verbindung des Gasleitungsabschnitts mit dem Kondensationsbehälter, insbesondere, falls es sich bei dem Kondensationsbehälter um einen weiteren Gasleitungsabschnitt handelt. Ferner können T-Stücke verwendet werden, welche an der Stelle des zweiten Gasauslasses im Übergangsbereich verjüngend oder düsenartig ausgebildet sind und somit eine vorteilhafte Düsenfunktion aufweisen.

In einer weiteren möglichen Ausgestaltung der Erfindung kann der Kondensationsbehälter ein weiterer Gasleitungsabschnitt mit einem Gaseinlass und einem Gasauslass sein. Dies ist insbesondere vorteilhaft, wenn der Gasleitungsabschnitt noch mit weiteren Komponenten, wie beispielsweise einem Drucksensor, verbunden wird, deren Funktionsweise unter Umständen durch sich in den Komponenten, also z.B. in dem Drucksensor, sammelndes Kondenswasser beeinträchtigt wird. Ist der weitere Gasleitungsabschnitt als Kondensationsbehälter ausgebildet, sammelt sich das Kondenswasser in dem Gasleitungsabschnitt und kann aus diesem direkt abgeführt werden ohne in die weiteren Komponenten, also z.B. den Drucksensor zu gelangen.

Vorzugsweise weist der Kondensationsbehälter einen Bereich mit einem Querschnitt auf, der um ein 10- bis 100000-faches, insbesondere ein 16-faches, 400-faches, 1600-faches oder 40000-faches größer ist als ein Querschnitt im Übergangsbereich vom zweiten Gasauslass zum Kondensationsbehälter. Des Weiteren ist es vorteilhaft, wenn ein Abstand zwischen diesen Bereichen mindestens 50 mm, insbesondere 50 bis 100 mm oder 75 mm beträgt.

Ein minimaler Durchmesser im Übergangsbereich vom zweiten Gasauslass zum Kondensationsbehälter beträgt vorzugsweise 0,5 bis 5 mm, insbesondere 1 mm oder 2,5 mm, und ein maximaler Durchmesser des Kondensationsbehälters beträgt vorzugsweise 20 bis 100 mm, insbesondere 50 mm oder 75 mm.

Der Gasleitungsabschnitt und/oder der weitere Gasleitungsabschnitt können einen Durchmesser von 5 bis 10 mm, insbesondere 6 mm oder 8 mm aufweisen. Der Gasleitungsabschnitt und/oder der weitere Gasleitungsabschnitt können als Schlauch oder Kunststoffrohr ausgebildet sein.

Weiterhin schließt die Erfindung ein pneumatisches, pulsatiles System für eine Membranpumpe eines Herzunterstützungssystems mit einem pneumatischen Antrieb, einem Betriebsgas und einer Gasleitung zum Führen des Betriebsgases, wobei die Gasleitung an die Membranpumpe anschließbar ist, ein. Das Erfindungsgemäße pulsatile System zeichnet sich dadurch aus, dass mit der Gasleitung eine vorstehend beschriebene Vorrichtung zum Abscheiden von Kondenswasser aus dem Betriebsgas verbunden ist.

Zur Erfindung gehört außerdem ein Herzunterstützungssystem mit einer Membranpumpe und einem mit der Membranpumpe verbundenen pneumatischen, pulsatilen System wie es vorstehend beschrieben ist.

Im Folgenden werden einige Beispiele einer erfindungsgemäßen Vorrichtung zur Abscheidung von Kondenswasser anhand von Figuren beschrieben. Dabei werden verschiedene erfindungswesentliche oder auch vorteilhafte weiterbildende Elemente im Rahmen dieser Beispiele genannt, wobei auch einzelne dieser Elemente als solche zur Weiterbildung der Erfindung - auch herausgelöst aus dem Kontext des jeweiligen Beispiels und weiterer Merkmale des jeweiligen Beispiels - verwendet werden können. Weiterhin werden in den Figuren für gleiche oder ähnliche Elemente gleiche oder ähnliche Bezugszeichen verwendet und deren Erläuterung daher teilweise weggelassen.

Es zeigen
- Figur 1: eine schematische Darstellung einer erfindungsgemäßen Vorrichtung zur Abscheidung von Kondenswasser gemäß einem ersten Ausführungsbeispiel,
- Figur 2: eine schematische Darstellung einer erfindungsgemäßen Vorrichtung zur Abscheidung von Kondenswasser gemäß einem zweiten Ausführungsbeispiel,
- Figur 3: einen Testaufbau zum Nachweis der Wirksamkeit der erfindungsgemäßen Vorrichtung und
- Figur 4: ein Diagramm zur Darstellung des zeitlichen Verlaufs der im Testaufbau aus Figur 3 abgeschiedenen Kondenswassermenge.

In den Figuren werden die folgenden Bezugszeichen verwendet:
- 1: Gasleitungsabschnitt
- 1a, 1b: erster, zweiter Gasleitungsabschnitt
- 2: Gaseinlass
- 3, 4: erster, zweiter Gasauslass
- 4a: Querschnitt des zweiten Gasauslasses
- 5: Kondensationsbehälter
- 5a: Querschnitt des Kondensationsbehälters
- 5b: Wandung
- 5d: Ende
- 6: Leitungsverbindungsstück
- 6a: Querschnitt des Leitungsverbindungsstücks
- 6b: Düse
- 7: Kondenswasser
- 8: Wasserdampf
- 9: T-Stück
- 9a, 9b: Verbindungsarme
- 9c: senkrechter Teil des T-Stücks
- 10: Stopfen
- 11: Pneumatischer Antrieb
- 12: Gasleitung zur Membranpumpe

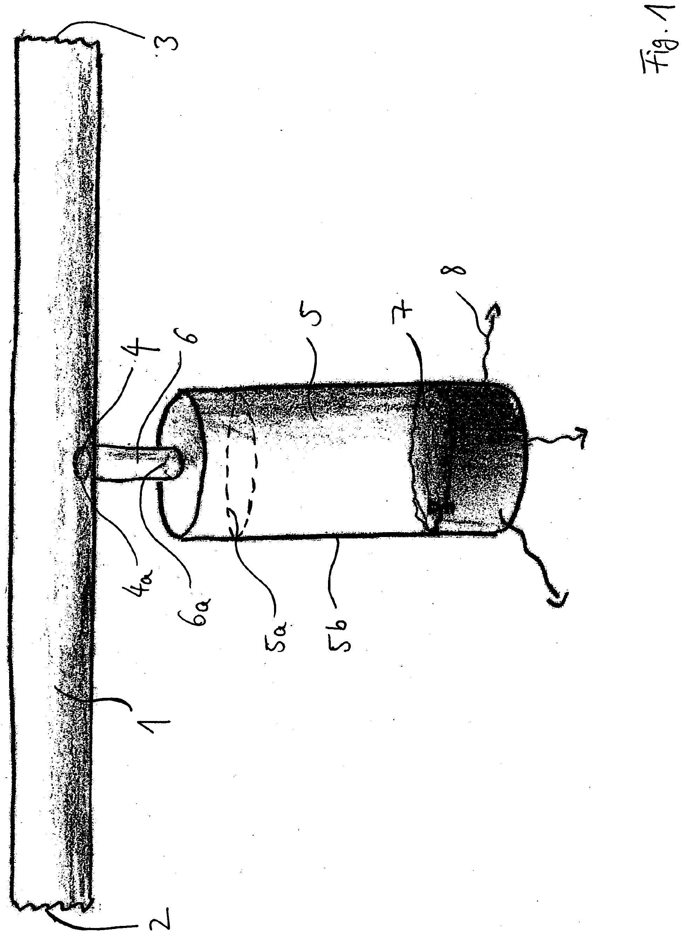

Figur 1 zeigt ein erstes Ausführungsbeispiel einer erfindungsgemäßen Vorrichtung zur Abscheidung von Kondenswasser aus einem Betriebsgas eines pneumatischen, pulsatilen Systems. Die Vorrichtung weist einen Gasleitungsabschnitt 1 mit einem Gaseinlass 2 und einem ersten Gasauslass 3 auf. Der Gasleitungsabschnitt 1 ist Teil einer Gasleitung des pneumatischen, pulsatilen Systems. Gaseinlassseitig ist die Vorrichtung mit einem Pneumatikantrieb (hier nicht gezeigt) verbindbar. Ein derartiger Pneumatikantrieb weist beispielsweise einen Zylinder auf, in welchem ein Kolben mittels eines Motors hin und her bewegt wird und dadurch periodisch einen Über- und Unterdruck in der Gasleitung erzeugt. Im Falle eines Herzunterstützungssystems mit einer Membranpumpe ist die Vorrichtung am ersten Gasauslass 3 mit der Membranpumpe verbindbar. Der Gasleitungsabschnitt 1 vermittelt somit den im Pneumatikantrieb erzeugten Über- und Unterdruck an die Membran der Membranpumpe. In etwa senkrecht zur Längsausdehnung des Gasleitungsabschnitts 1 weist der Gasleitungsabschnitt 1 einen zweiten Gasauslass 4 mit einem Querschnitt 4a auf. Mit dem zweiten Gasauslass 4 ist ein Kondensationsbehälter 5 mit einem konstanten Querschnitt 5a mittels eines Leitungsverbindungsstücks 6 mit Querschnitt 6a verbunden. Der Querschnitt 6a des Leitungsverbindungsstücks 6 entspricht dabei dem Querschnitt 4a des zweiten Gasauslasses 4a. Der Querschnitt 5a des Kondensationsbehälters 5 ist deutlich größer als der Querschnitt 6a des Leitungsverbindungsstücks 6. Der Kondensationsbehälter 5 weist ferner eine Wandung 5b auf, die ein halbdurchlässiges Material enthält. Dieses Material ist durchlässig für Wasser und/oder Wasserdampf und undurchlässig für das Betriebsgas.

Die Wirkungsweise der erfindungsgemäßen Vorrichtung gemäß dem ersten Ausführungsbeispiel kann qualitativ in etwa wie folgt beschrieben werden: Strömt das Betriebsgas durch den Gasleitungsabschnitt 1, gelangt ein Teil des Betriebsgases durch den zweiten Gasauslass 4 und das Leitungsverbindungsstück 6 in den Kondensationsbehälter 5. Beim Übergang vom Leitungsverbindungsstück 6 zum Kondensationsbehälter 5 trifft das Betriebsgas auf eine Querschnittvergrößerung, nämlich vom kleineren Querschnitt 6a zum deutlich größeren Querschnitt 5a. Dies führt zu einer raschen Expansion des Betriebsgases im Kondensationsbehälter und zu einer deutlichen Abkühlung des Betriebsgases. Hierdurch kondensiert der im Betriebsgas enthaltene Wasserdampf im Kondensationsbehälter zu Wasser 7. Das kondensierte Wasser 7 benetzt den unteren Teil der Wandung 5b des Kondensationsbehälters 5 und wird über die wasserdurchlässige Wandung 5b des Kondensationsbehälters als Wasserdampf oder Wasser aus dem Kondensationsbehälter 5 und somit aus dem pneumatischen, pulsatilen System abgeführt.

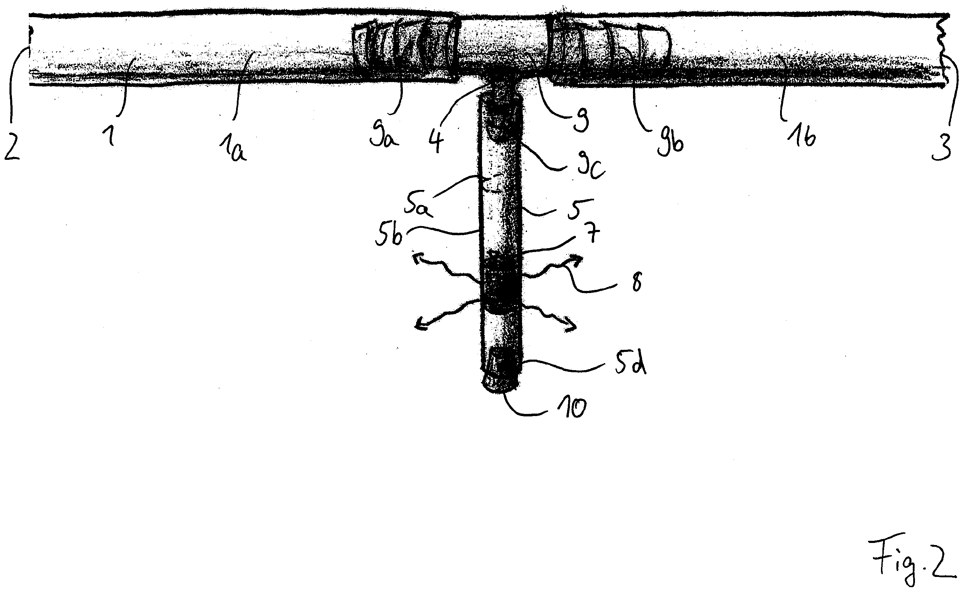

Figur 2 zeigt ein zweites Ausführungsbeispiel einer erfindungsgemäßen Vorrichtung zur Abscheidung von Kondenswasser aus einem pneumatischen, pulsatilen System. In diesem Ausführungsbeispiel ist der Gasleitungsabschnitt 1 in einen ersten 1a und einen zweiten Gasleitungsabschnitt 1b gegliedert. Der Gasleitungsabschnitt 1 weist einen Gaseinlass 2 und einen ersten Gasauslass 3 auf. Der erste 1a und der zweite Gasleitungsabschnitt 1b sind über ein T-Stück 9 miteinander verbunden. Das T-Stück 9 weist zwei in einer Linie verlaufende Verbindungsarme 9a und 9b auf, wobei der Verbindungarm 9a gasauslassseitig in den ersten Gasleitungsabschnitt 1a eingesteckt ist und der Verbindungsarm 9b gaseinlassseitig in den zweiten Gasleitungsabschnitt 9b eingesteckt ist. Über das T-Stück 9 kann das Betriebsgas vom Gaseinlass 2 zum ersten Gasauslass 3 bzw. in umgekehrter Richtung strömen. Das T-Stück 9 weist weiterhin einen senkrechten Teil 9c auf, welcher im Wesentlichen mittig zwischen den Verbindungsarmen 9a und 9b angeordnet ist und einen zweiten Gasauslass 4 bildet. Der senkrechte Teil 9c ist derart ausgerichtet, dass er eine Strömung des Betriebsgases durch den zweiten Gasauslass 4 im Wesentlichen senkrecht zur Längsausdehnung des Gasleitungsabschnitts 1 ermöglicht. Der senkrechte Teil 9c ist ferner mit einem weiteren Gasleitungsabschnitt 5 verbunden, welcher einen Kondensationsbehälter bildet. Der weitere Gasleitungsabschnitt 5 ist an seinem dem Gasleitungsabschnitt 1 abgewandten Ende 5d mit einem Stopfen 10 verschlossen. Alternativ ist es denkbar, anstelle des Stopfens 10 an das Ende 5d einen Drucksensor anzuschließen. Der senkrechte Teil 9c weist ausgehend vom Gasauslass 4 einen sich verjüngenden Querschnitt auf und wirkt daher wie eine Düse, welche den Kondensationseffekt im weiteren Gasleitungsabschnitt 5 verstärken kann. Der Querschnitt 5a des Kondensationsbehälters ist wie im ersten Ausführungsbeispiel deutlich größer als der Querschnitt am zweiten Gasauslass 4 und im übrigen Teil des senkrechten Teils 9c. Der weitere Gasleitungsabschnitt weist ferner eine Wandung 5b auf, die ein halbdurchlässiges Material enthält. Dieses Material ist durchlässig für Wasser und/oder Wasserdampf und undurchlässig für das Betriebsgas.

Die Wirkungsweise der Vorrichtung des zweiten Ausführungsbeispiels kann qualitativ in etwa wie folgt beschrieben werden: Ein Teil des durch den Gasleitungsabschnitt 1 strömenden Betriebsgases gelangt durch den zweiten Gasauslass 4 in den senkrechten Teil 9c des T-Stücks 9. Aufgrund des sich verjüngenden Querschnitts des senkrechten Teils 9c wird dieser Teil des Betriebsgases im senkrechten Teil 9c komprimiert. Durch diese Komprimierung kann ein Teil des in dem Betriebsgas gebundenen Wasserdampfes in Form von Wasser ausfallen und in den weiteren Gasleitungsabschnitt 5 als Kondensationsbehälter gelangen. Am Ende des senkrechten Teils 9c des T-Stücks 9, wenn das Betriebsgas in den weiteren Gasleitungsabschnitt 5 gelangt, trifft das Betriebsgas auf eine Querschnittvergrößerung, wodurch sich dieses rasch ausdehnt und abkühlt. Durch die Abkühlung kondensiert ein weiterer Teil des im Betriebsgas gebundenen Wasserdampfes und wird im weiteren Gasleitungsabschnitt 5 gesammelt. Über die halbdurchlässige Wandung 5b kann das gesammelte Kondenswasser 7 als Wasserdampf 8 oder Wasser aus dem weiteren Gasleitungsabschnitt 5 und somit aus dem pneumatischen, pulsatilen System abgeführt werden, bevor es in einen gegebenenfalls am Ende 9d angeschlossenen Drucksensor gelangen kann.

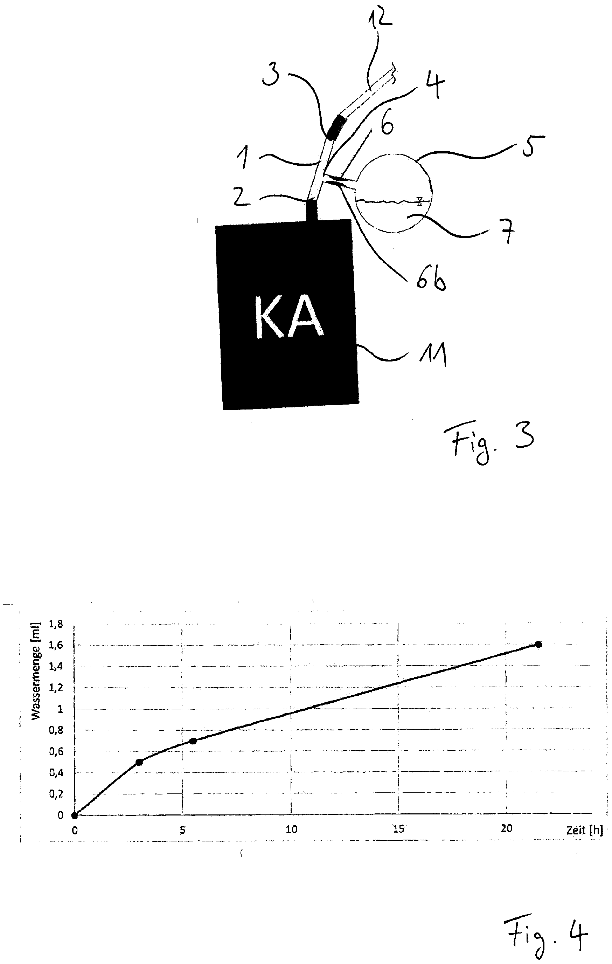

Figur 3 zeigt schematisch einen Testaufbau zum Nachweis der Wirksamkeit der erfindungsgemäßen Vorrichtung in einem Herzunterstützungssystem mit einer Membranpumpe. Der gezeigte Testaufbau umfasst lediglich den Antriebsstrang des Herzunterstützungssystems. Der Gasleitungsabschnitt 1 ist an seinem Gaseinlass 2 mit einem pneumatischen Antrieb 11 verbunden. An seinem ersten Gasauslass 3 ist eine Gasleitung 12, mit welcher die Membranpumpe verbunden ist, angeschlossen. Der erste Gasleitungsabschnitt 1 zusammen mit der Gasleitung 12 werden auch als Antriebsschlauch bezeichnet. Am zweiten Gasauslass 4 ist ein Kondensationsbehälter 5 über ein Leitungsverbindungsstück 6 angeschlossen. Der Kondensationsbehälter 5 ist hier als sphärischer Behälter dargestellt. Das Leitungsverbindungsstück 6 weist eine Düse 6b auf. Ferner dient Luft als Betriebsgas im Testaufbau. Bei jedem Hub des im pneumatischen Antrieb 11 arbeitenden Kolbens wird etwas feuchte Luft durch die Düse 6b gepresst und anschließend expandiert. Bei diesem Vorgang kondensiert das in der Luft gespeicherte Wasser und sammelt sich im Kondensationsbehälter 5. So ist eine Entfeuchtung der Luft im Antriebsschlauch 1, 12 möglich.

Für den Test wird der pneumatische Antrieb 11 an einem Kreislaufmodell mit einer Membranpumpe mit 80 ml Hubvolumen betrieben. Nach einer Warmlaufphase von ca. 2h wird über eine Spritze destilliertes Wasser in den Antriebsschlauch 1, 12 gegeben. Im verwendeten Testaufbau wird die Düse 6b des Leitungsverbindungsstücks von einem Luer-Adapter gebildet. Der Kondensationsbehälter 5 wird von einer mit dem Luer-Adapter verbundenen Spritze gebildet, wobei die Position des Kolbens der Spritze fixiert wurde.

Zur Bestimmung der abgeschiedenen Kondenswassermenge wird der Kondensationsbehälter vom Luer-Adapter entfernt und gewogen. Das Gewicht vor Testbeginn betrug 40,0 g. Der zeitliche Verlauf der abgeschiedenen Menge ist in Figur 4 dargestellt.

Nach einer Zeit von 21,5 h wurde der Test beendet. Die abgeschiedene Wassermenge betrug 1,6 ml, wobei der Antriebschlauch 1, 12 völlig trocken war. Somit liegt die Abscheiderate bei > 1,8 ml pro Tag.

Die Wirksamkeit der Kondenswasserabscheidung über eine Düse und einen angeschlossenen Kondensationsbehälter ist damit bestätigt.

## Patentansprüche

1. Vorrichtung zum Abscheiden von Kondenswasser aus einem Betriebsgas eines pneumatischen, pulsatilen Systems, wobei die Vorrichtung
einen Gasleitungsabschnitt mit einem Gaseinlass und einem ersten und einem zweiten Gasauslass aufweist,
**dadurch gekennzeichnet, dass**
die Vorrichtung einen Kondensationsbehälter zum Extrahieren des Kondenswassers aus dem Betriebsgas umfasst, der mit dem zweiten Gasauslass verbunden ist, wobei ein Übergangsbereich vom zweiten Gasauslass zum Kondensationsbehälter eine Querschnittvergrößerung aufweist, und
der Kondensationsbehälter ein Mittel zum Abführen des extrahierten Kondenswassers aufweist.

2. Vorrichtung nach dem vorhergehenden Anspruch, **dadurch gekennzeichnet, dass** der Kondensationsbehälter als Mittel zum Abführen des extrahierten Kondenswassers ein wasserdurchlässiges Material, insbesondere einen semipermeablen Kunststoff, ein Fluorpolymer und/oder ein Sintermetall aufweist.

3. Vorrichtung nach einem der vorhergehenden Ansprüche, wobei der Übergangsbereich vom zweiten Gasauslass zum Kondensationsbehälter vor der Querschnittvergrößerung eine Querschnittverengung aufweist.

4. Vorrichtung nach einem der vorhergehenden Ansprüche, wobei zwischen dem zweiten Gasauslass und dem Kondensationsbehälter ein Leitungsverbindungsstück angeordnet ist, wobei ein minimaler Querschnitt des Leitungsverbindungsstücks kleiner ist als ein minimaler Querschnitt des zweiten Gasauslasses und/oder als ein maximaler Querschnitt des Kondensationsbehälters.

5. Vorrichtung nach dem vorhergehenden Anspruch, **dadurch gekennzeichnet, dass** der Querschnitt des Leitungsverbindungsstücks zwischen dem zweiten Gasauslass und dem Kondensationsbehälter konstant ist.

6. Vorrichtung nach Anspruch 4, **dadurch gekennzeichnet, dass** der Leitungsverbindungsabschnitt eine Düse mit einem sich vom zweiten Gasauslass zum Kondensationsbehälter hin verengenden Querschnitt aufweist.

7. Vorrichtung nach einem der vorhergehenden Ansprüche, **dadurch gekennzeichnet, dass** der Gasleitungsabschnitt einen ersten Gasleitungsabschnitt und einen zweiten Gasleitungsabschnitt aufweist, welche über in einer Linie verlaufende Verbindungsarme eines T-Stücks miteinander verbunden sind, und der zweite Gasauslass von dem Teil des T-Stücks gebildet wird, welcher weitgehend senkrecht zu den Verbindungsarmen abgezweigt ist.

8. Vorrichtung nach einem der vorhergehenden Ansprüche, wobei der Kondensationsbehälter ein weiterer Gasleitungsabschnitt mit einem Gaseinlass und einem Gasauslass ist.

9. Vorrichtung nach dem vorhergehenden Anspruch, **dadurch gekennzeichnet, dass** der Gasauslass des weiteren Gasleitungsabschnittes mit einem Drucksensor verbunden ist.

10. Vorrichtung nach einem der vorhergehenden Ansprüche, **dadurch gekennzeichnet, dass** der Kondensationsbehälter einen Bereich mit einem Querschnitt aufweist, der um ein 10- bis 100000-faches, insbesondere ein 16-faches, 400-faches, 1600-faches oder 40000-faches größer ist als ein Querschnitt im Übergangsbereich vom zweiten Gasauslass zum Kondensationsbehälter und dass ein Abstand zwischen diesen Bereichen mindestens 50 mm, insbesondere 50 bis 100 mm oder 75 mm beträgt.

11. Vorrichtung nach einem der vorhergehenden Ansprüche, **dadurch gekennzeichnet, dass** ein minimaler Durchmesser im Übergangsbereich vom zweiten Gasauslass zum Kondensationsbehälter 0,5 bis 5 mm, insbesondere 1 mm oder 2,5 mm beträgt und ein maximaler Durchmesser des Kondensationsbehälters 20 bis 100 mm, insbesondere 50 mm oder 75 mm beträgt.

12. Vorrichtung nach einem der vorhergehenden Ansprüche, **dadurch gekennzeichnet, dass** der Gasleitungsabschnitt und/oder der weitere Gasleitungsabschnitt einen Durchmesser von 5 bis 10 mm, insbesondere 6 mm oder 8 mm aufweist.

13. Vorrichtung nach einem der vorhergehenden Ansprüche, **dadurch gekennzeichnet, dass** der Gasleitungsabschnitt und/oder der weitere Gasleitungsabschnitt ein Schlauch oder ein Kunststoffrohr ist.

14. Pneumatisches, pulsatiles System für eine Membranpumpe eines Herzunterstützungssystems mit einem pneumatischen Antrieb, einem Betriebsgas und einer Gasleitung zum Führen des Betriebsgases, wobei die Gasleitung an die Membranpumpe anschließbar ist, **dadurch gekennzeichnet, dass** mit der Gasleitung eine Vorrichtung nach einem der vorhergehenden Ansprüche verbunden ist.

15. Herzunterstützungssystem mit einer Membranpumpe und einem mit der Membranpumpe verbundenen pneumatischen, pulsatilen System nach dem vorhergehenden Anspruch.
